(19) Europäisches Patentamt
European Patent Office
Office européen des brevets

(11) Publication number: **0 137 979**
**B1**

# (12) EUROPEAN PATENT SPECIFICATION

(45) Date of publication of patent specification: **26.10.88**

(21) Application number: **84110036.5**

(22) Date of filing: **23.08.84**

(51) Int. Cl.[4]: **C 07 D 239/26**, C 07 D 239/42, C 07 D 239/52, C 07 D 237/08, C 07 D 237/12, C 07 D 241/12, C 07 D 241/16, A 61 K 31/505, A 61 K 31/50, A 61 K 31/495

## (54) Diazine-ethenylphenyl oxamic acids and esters and salts thereof.

(30) Priority: **01.09.83 US 528522**

(43) Date of publication of application:
**24.04.85 Bulletin 85/17**

(45) Publication of the grant of the patent:
**26.10.88 Bulletin 88/43**

(84) Designated Contracting States:
**AT BE CH DE FR GB IT LI LU NL SE**

(56) References cited:
DE-A-2 362 409
DE-A-2 509 457
DE-A-2 525 226
US-A-3 714 165
US-A-4 054 666
US-A-4 238 496

**The file contains technical information submitted after the application was filed and not included in this specification**

(73) Proprietor: **Boehringer Ingelheim Ltd.**
**90 East Ridge P.O. Box 368**
**Ridgefield, Conn 06877 (US)**

(72) Inventor: **Grozinger, Karl G. Dr.**
**171 High Ridge Avenue**
**Ridgefield Connecticut 06877 (US)**
Inventor: **Oliver, James T. Dr.**
**116 North Street**
**Middlebury Connecticut 06762 (US)**

(74) Representative: **Milnes, Rodger et al**
**Boehringer Ingelheim Zentrale GmbH ZA**
**Patente Postfach 200**
**D-6507 Ingelheim am Rhein (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 137 979 B1

**Description**

This invention relates to novel diazine-ethenylphenyl oxamic acids and esters and salts thereof, to methods of preparing these compounds, to pharmaceutical compositions containing them as active ingredients, and to methods of using them for the treatment of immunological, inflammatory and allergic disorders.

Cromoglycate, normally administered as the sodium salt, is a potent and useful antiallergic, commonly prescribed for the treatment of bronchial asthma. Cromoglycate has for many years been accepted as an effective bronchodilator when given by inhalation as a solid. However, it is known to have certain disadvantages; for instance, it is not active when given orally, which warrants a search for new orally active antiallergics.

A number of oxamate derivatives have been disclosed in the patent and scientific literature. Illustrative of such prior art are the following:

(a) 4-Substituted thiazol-2-oxamic acids, U.S. Patent 4,239,496,

(b) N,N'-(phenylene)dioxamic acid and its derivatives, German Offenlegungsschrift 2,362,409,

(c) N-phenyloxamic acid derivatives, which are stated to be useful for prophylaxis against allergics and anaphylactic reactions, German Offenlegungsschrift 2,525,226,

(d) Aromatic and heterocyclic oxamic acid derivatives, which are stated to be suitable for the treatment of immediate hypersensitivity reactions, U.S. Patent 4,054,666, and

(e) N-(2-Thienyl)-oxamic acid derivatives, which are stated to be useful for prophylaxis against allergics and anaphylactic reaction, German Offenlegungsschrift 2,509,457.

The prior art, however, does not disclose diazineethenylphenyl oxamic acids or their esters or salts.

The Invention

More particularly, the present invention relates to novel diazine-ethenylphenyl oxamic acids represented by the formula

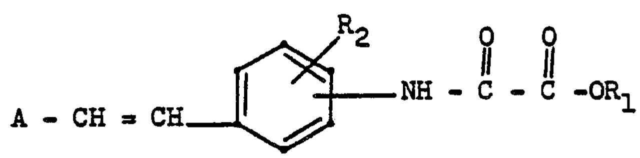 (I)

wherein

$R_1$ is hydrogen or alkyl of 1 to 4 carbon atoms;

$R_2$ is hydrogen, methyl, hydroxyl, alkoxy of 1 to 4 carbon atoms, di(alkyl of 1 to 2 carbon atoms)amino-(alkoxy of 1 to 4 carbon atoms) or —HN—CO—CO—$OR_1$;

A is

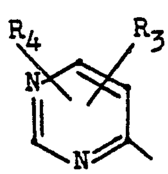 , 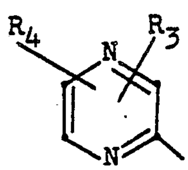 or 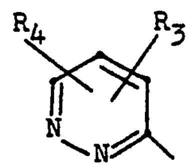

$R_3$ is hydrogen, methyl, alkoxy of 1 to 4 carbon atoms, hydroxyl, amino, alkanoyloxy of 1 or 2 carbon atoms, di(alkyl of 1 or 2 carbon atoms)amino-(alkoxy of 1 to 4 carbon atoms) or acetamido; and

$R_4$ is hydrogen, amino, alkoxy of 1 to 4 carbon atoms, halogen or —HN—CO—CO—$OR_1$;

and, when $R_1$ is hydrogen, non-toxic, pharmacologically acceptable salts thereof, especialy their alkali metal and primary or secondary amine salts.

A preferred subgenus is constituted by those compounds of the formula I

wherein

$R_1$ is hydrogen or ethyl,

$R_2$ is hydrogen,

A is

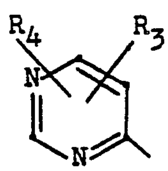 , 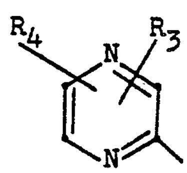 or 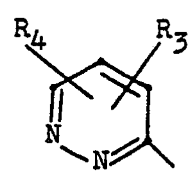

$R_3$ is hydrogen or methyl, and

$R_4$ is hydrogen, or —HN—CO—CO—$OR_1$,

and non-toxic, pharmacologically acceptable salts thereof.

The compounds embraced by formula I may be prepared by reacting a [2-(aminophenyl)ethenyl]-diazine of the formula

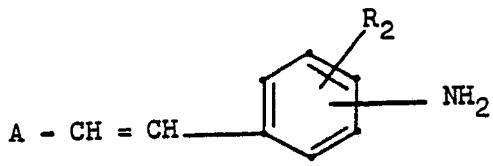

(II)

wherein A and $R_2$ have the same meanings as in formula I, with an oxalate halide, preferably the chloride, or with a dialkyl oxalate, optionally followed by hydrolysis of the ester group.

The reaction is preferably carried out by dissolving or suspending a compound of the formula II, or an acid addition salt thereof, in an inert liquid medium, and admixing the oxalic acid derivative slowly, preferably dropwise with the solution or suspension. Examples of suitable inert liquid media are benzene, toluene, xylene, methylene chloride, dimethylformamide or tetrahydrofuran. In addition, it is preferred to add an organic base, such as pyridine or triethylamine, to the reaction mixture to neutralize the acid release by the reaction. Since the reaction is strongly exothermic, the oxalic acid derivative should be added slowly and, if necessary, while cooling.

Since most [2-(aminophenyl)ethyl]diazines are sparsely soluble, it is of advantage to let the reaction mixture stand for an extended period of time, for instance overnight, with or without stirring, before isolating the reaction product.

The reaction mixture is worked up in conventional manner, that is, by evaporating the inert liquid medium, extracting the residue with a suitable solvent or solvent mixture, such as ether, ethyl acetate, chloroform, hexane or mixtures of any two or more of these, purifying the extract solution, evaporating the solvent, and recrystallizing the residue. In some cases further purification by column chromatography is of advantage.

If it is desired to obtain an end product of the formula I wherein $R_1$ is hydrogen, the ester group is removed by hydrolysis with a basic or acid catalyst. Suitable such catalysts are strong bases such as sodium hydroxide or potassium hydroxide, or mineral acids such as hydrochloric acid, sulfuric acid or phosphoric acid.

The starting compounds of the formula II may be prepared by a 2-step process which comprises

(a) Reaction of a nitrobenzaldehyde with a methyldiazine (pyrimidine, pyrazine or pyridazine) in a suitable solvent such as acetic anhydride, formic acid or the like to produce an intermediate [2-(nitrophenyl)ethenyl]diazine, pursuant to the following reaction scheme:

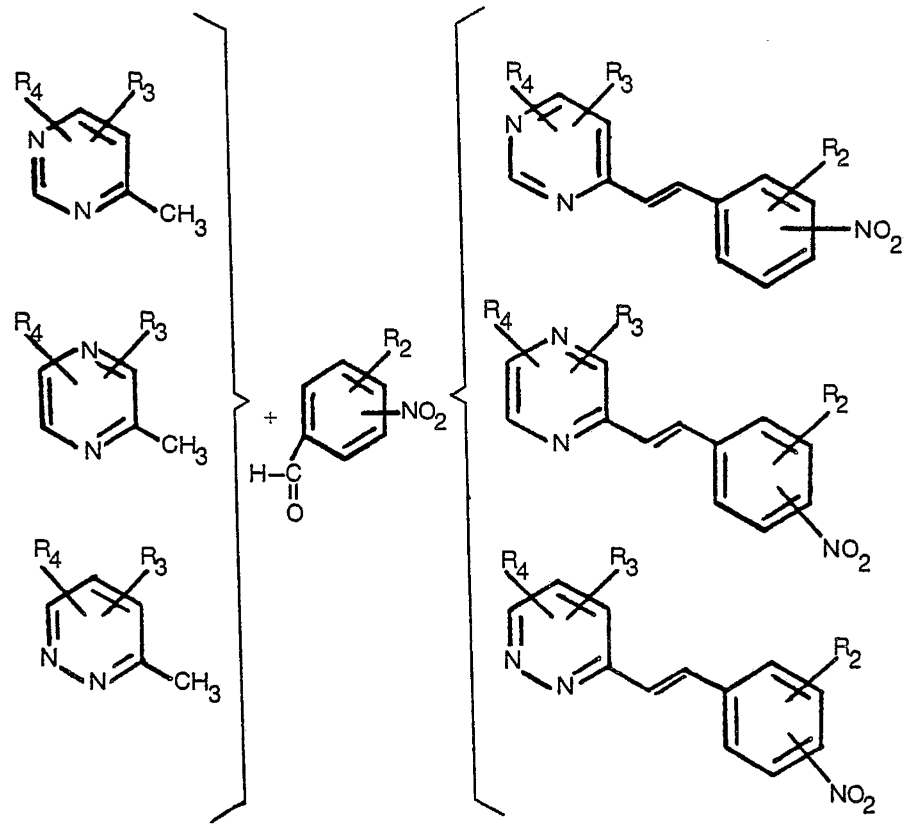

(b) Reduction of the [2-(nitrophenyl)ethenyl]diazine to the corresponding amine with a metal or metal salt, such as iron, tin or zinc, in an aqueous acid according to A. J. Bechamp, Ann. Chim. Phys. [3] *42*, 186, (1854).

The preparation of some [2-(nitrophenyl)ethenyl]diazine derivatives is described in the literature. For example, 4-[2-(p-nitrophenyl)ethenyl]-2-aminopyrimidine

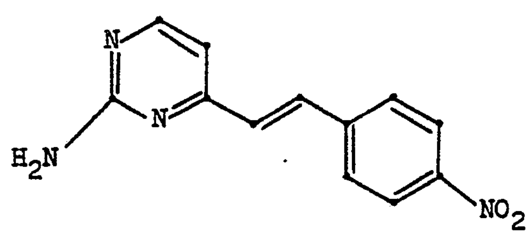

may be prepared according to the procedure disclosed in C.A. 62: 10448-c (Japanese Patent 19652, 1964). Similarly, the compounds

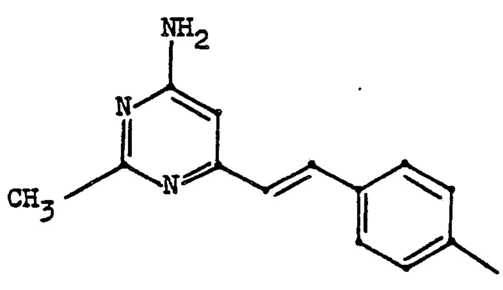

and

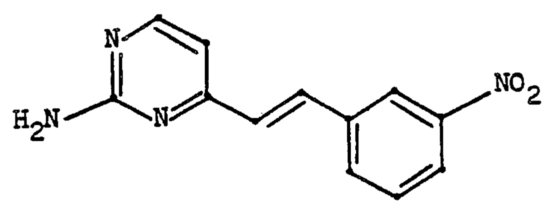

may be prepared by the general procedures described in J. Med. Chem. 1290 (1970), J. Chem. Soc. C 1343 (1967) and J. Pharm. Soc. Jap. *72*, 909 (1953).

Using the above-indicated method, the following end products of the formula I, optionally in the form of their salts or lower alkyl esters, can be prepared:

Ethyl 2-(4-pyrimidinyl)ethenylphenyl-4 oxamate;
2-(4-Pyrimidinyl)ethenylphenyl-4 oxamic acid ethanolamine salt;
Ethyl 2-(4-pyrimidinyl)ethenylphenyl-3 oxamate;
2-(4-Pyrimidinyl)ethenylphenyl-3 oxamic acid ethanolamine salt;
Ethyl 2-(4-pyrimidinyl)ethenylphenyl-2 oxamate;
2-(4-Pyrimidinyl)ethenylphenyl-2 oxamic acid ethanolamine salt;
Ethyl 2-(4-pyrimidinyl)ethenyl-2-hydroxyphenyl-4 oxamate;
Ethyl 2-(4-pyrimidinyl)ethenyl-5-chlorophenyl-3 oxamate;
Ethyl 2-(4-pyrimidinyl)ethenyl-3-N,N-dimethylamino-ethoxyphenyl-4 oxamate;
Ethyl 2-(4-pyrimidinyl)ethenyl-4-N,N-dimethylamino-ethoxyphenyl-3 oxamate;
Diethyl 2-(4-pyrimidinyl)ethenylphenyl-2,4-dioxamate;
Ethyl 2-(2,6-diethoxypyrimidin-4-yl)ethenylphenyl-4 oxamate;
Ethyl 2-(2-methyl-6-aminopyrimidin-4-yl)ethenylphenyl-4 oxamate;
Ethyl 2-(6-methylpyrimidin-4-yl)ethenylphenyl-4 oxamate;
Ethyl 2-(2-acetamidopyrimidin-4-yl)ethenylphenyl-3 oxamate;
2-(2-Acetamidopyrimidin-4-yl)ethenylphenyl-3 oxamic acid tromethane salt;
Ethyl 2-(3-pyridazinyl)ethenylphenyl-4 oxamate;
2-(3-Pyridazinyl)ethenylphenyl-4 oxamic acid ethenolamine salt;
Ethyl 2-(2-pyrazinyl)ethenylphenyl-4 oxamate;
2-(2-Pyrazinyl)ethenylphenyl-4 oxamic acid sodium salt;
Ethyl 2-(2-pyrazinyl)ethenylphenyl-3 oxamate;
2-(2-Pyrazinyl)ethenylphenyl-3 oxamic acid ethanolamine salt;
Ethyl 2-(2-methyl-6-ethoxyoxalylaminopyrimidin-4-yl)ethenylphenyl-4 oxamate;
2-(2-Methyl-6-carboxycarbonylaminopyrimidin-4-yl)ethenylphenyl-4 oxamic acid hydrate diethanolamine salt;
Ethyl 2-(2-methyl-6-ethoxyoxalylaminopyrimidine-4-yl)ethenylphenyl-3 oxamate hemihydrate;
2-(2-Methyl-6-carboxycarbonylaminopyrimidin-4-yl))ethenylphenyl-3 oxamic acid di-tromethane salt;
Ethyl 2-(2-ethoxyoxalylaminopyrimidin-4-yl)ethenylphenyl-3 oxamate;
2-(2-Carboxycarbonylaminopyrimidin-4-yl)ethenylphenyl-3 oxamic acid tetra-sodium salt di-hydrate;
Ethyl 2-(2-ethoxyoxalylaminopyrimidin-4-yl)ethenylphenyl-4 oxamate;
Ethyl 2-(3-chloropyrazin-2-yl)ethenylphenyl-4 oxamate;
2-(3-Chloropyrazin-2-yl)ethenylphenyl-4 oxamic acid ethanolamine salt;
Ethyl 2-(3-ethylpyrazin-2-yl)ethenylphenyl-4 oxamate;
2-(3-Ethylpyrazin-2-yl)ethenylphenyl-4 oxamic acid ethanolamine salt;
Ethyl 2-(6-chloropyridazin-3-yl)ethenylphenyl-4 oxamate;
2-(6-Chloropyridazin-3-yl)ethenylphenyl-4 oxamic acid ethanolamine salt;
Ethoxyethyl 2-(2-pyrazinyl)ethenylphenyl-4 oxamate;
Ethoxyethyl 2-(2-methyl-6-aminopyrimidin-4-yl)ethenylphenyl-4 oxamate hemihydrate.

The following examples illustrate the present invention.

## Example 1

Ethyl 2-(4-pyrimidinyl)ethenylphenyl-4 oxamate

1.2 g of ethyl oxalyl chloride were added dropwise to a suspension of 1.3 g of 4-[2-(p-aminophenyl)-ethenyl]pyrimidine in 30 ml of methylene chloride containing 1.6 ml of pyridine, and the mixture was stirred overnight at room temperature. Thereafter, the reaction mixture was washed first with an aqueous sodium bicarbonate solution, then with water and then with a saturated aqueous sodium chloride solution, dried over magnesium sulfate and finally evaporated to dryness. The residue was recrystallized from chloroform and petroleum ether, yielding 1.3 g (66.3% of theory) of the title compound which had a melting point of 171—173°C.

The starting compound was prepared as follows: 24.2 g of p-nitrobenzaldehyde, 15.5 g of 4-methylpyrimidine and 16.7 g of acetic acid anhydride were mixed at room temperature, and the mixture was heated at 120°C for 5 hours. After cooling, the mixture was poured into 500 ml of water, and the aqueous mixture was extracted several times with chloroform. The combined chloroform extracts were dried over magnesium sulfate and concentrated until crystals separated. Addition of ether gave 28 g (77% of theory) of 4-[2-(p-nitrophenyl)ethenyl]pyrimidine, m.p. 213—215°C.

100 ml of 4N hydrochloric acid were added dropwise to a mixture of 28 g of 4-[2-(p-nitrophenyl)-ethenyl]pyrimidine, 250 ml of ethanol and 28 g of iron filings. The reaction temperature increased to 65°C, and the mixture was stirred at that temperature for two hours. Water was added, followed by 200 ml of an aqueous 30% potassium hydroxide solution, and 1 liter of chloroform. The mixture was filtered through celite, and the chloroform layer was separated, dried over sodium sulfate and then concentrated.

Addition of ether gave 10.3 g (42.4% of theory) of 4-[2-(p-aminophenyl)ethenyl]pyrimidine, m.p. 227—230°C, which was used as the starting compound without further purification.

Example 2

2-(4-Pyrimidinyl)ethenylphenyl-4 oxamic acid and its ethanolamine salt

13.5 ml of a 1N sodium hydroxide solution was added dropwise to a suspension of 4 g of ethyl 4-[2-(4-pyrimidinyl)ethenyl]phenyl oxamate (see Example 1) in 50 ml water and 50 ml ethanol while vigorously stirring until a clear solution was formed. The resulting solution was acidified with 2N hydrochloric acid, and the precipitate formed thereby was filtered off. The filter cake was dried to give 2 g of 2-(4-pyrimidinyl)ethenylphenyl-4 oxamic acid, m.p. 214—216°C.

The acid was suspended in a mixture of 50 ml N,N-dimethyl formamide and 1 g of ethanolamine, and the precipitate formed thereby was filtered off and washed with ether. 1.3 g (29% of theory) of the ethanolamine salt of the acid compound, m.p. 202—205°C, were obtained.

Example 3

Ethyl 2-(2-ethoxyoxalylaminopyrimidin-4-yl)ethenylphenyl-4 oxamate

19 ml of ethyl oxalyl chloride were added dropwise to a solution of 13.5 g of 4-[2-(p-aminophenyl)-ethenyl]-2-aminopyrimidine in 75 ml of dry pyridine. The reaction mixture was then stirred for 12 hours at room temperature; the progress of the reaction was periodically checked by thin-layer chromatography. After the completion of the reaction, the reaction mixture was poured into ice water. The crude product was extracted with chloroform, dried and purified on a silica-gel column. After recrystallization from chloroform and ether, 9.0 g (34% of theory) of ethyl 2-[2-(ethoxyoxalylaminopyrimidin-4-yl)ethenyl]phenyl-4 oxamate, m.p. 191—193°C, were obtained.

The starting compound was prepared as follows:

A solution of 21.8 g of 2-amino-4-methylpyrimidine and 30.2 g of p-nitrobenzaldehyde in 45 ml of formic acid was refluxed for 24 hours. After cooling, the reaction mixture was poured into 1 liter of water, and the aqueous mixture was neutralized with a 5N sodium hydroxide solution. The crude product was extracted with chloroform, and the extract was dried over sodium sulfate and concentrated to dryness. The crude product was purified on a silica gel column to give 27.8 g (57% of theory) of 4-[2-(p-nitrophenyl)-ethenyl]-2-aminopyrimidine, m.p. 214—216°C, which was used for the next step without purification.

100 ml of 4N hydrochloric acid were added dropwise to a stirred mixture of 21.8 g of 4-[2-(p-nitrophenyl)-ethenyl]-2-aminopyrimidine, 250 ml of ethanol and 22 g of iron filing. During the addition the reaction temperature increased to 65°C, and stirring was continued for two hours at 65°C. Water was added, followed by 200 ml of an aqueous 30% sodium hydroxide solution and 1 liter of chloroform. The chloroform layer was then separated and dried over sodium sulfate. After evaporation and addition of ether, 13.5 g (71.7% of theory) of 4-[2-(p-aminophenyl)-ethenyl]-2-aminopyrimidine were obtained, which was used as the starting compound without further purification.

Using the appropriate starting compounds and procedures analogous to those described in the preceding examples, the following compounds of the formula

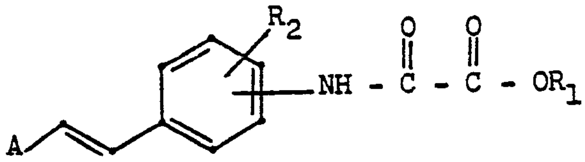

were also prepared:

| Example No. | A | $R_2$ | $NHCOCOOR_1$ | m.p. °C |
|---|---|---|---|---|
| 4 | 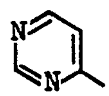 | H | 3-$NHCOCOOC_2H_5$ | 127-128 |
| 5 | 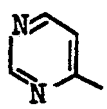 | H | 3-NHCOCOOH Ethanolamine salt | 161-163 |
| 6 | 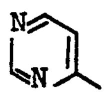 | H | 2-$NHCOCOOC_2H_5$ | 105-110 |
| 7 | 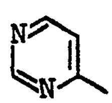 | H | 2-NHCOCOOH Ethanolamine salt | 151-153 |
| 8 | 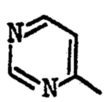 | 2-OH | 4-$NHCOCOOC_2H_5$ | 145-147 |
| 9 | 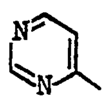 | 5-Cl | 3-$NHCOCOOC_2H_5$ | 147-148 |
| 10 | 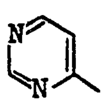 | 3-$OCH_2CH_2N(CH_3)_2$ | 4-$NHCOCOOC_2H_5$ | 156-159 |
| 11 | 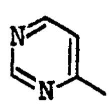 | 4-$OCH_2CH_2N(CH_3)_2$ | 3-$NHCOCOOC_2H_5$ sesquihydrochloride | 176-178 |
| 12 | 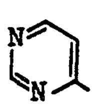 | 2-$NHCOCOOC_2H_5$ | 4-$NHCOCOOC_2H_5$ | 135-137 |
| 13 | 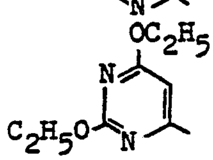 | H | 4-$NHCOCOOC_2H_5$ | 156-159 |

| Example No. | A | $R_2$ | $NHCOCOOR_1$ | m.p. °C |
|---|---|---|---|---|
| 14 | 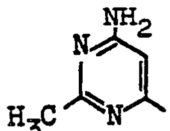 | H | 4-$NHCOCOOC_2H_5$ | 267-269 |
| 15 | 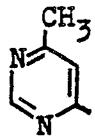 | H | 4-$NHCOCOOC_2H_5$ | 149-151 |
| 16 | 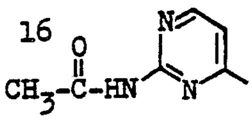 | H | 3-$NHCOCOOC_2H_5$ | 234-236 |
| 17 | 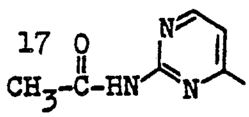 | H | 3-NHCOCOOH Tromethane salt | 195-197 |
| 18 | 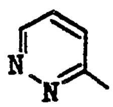 | H | 4-$NHCOCOOC_2H_5$ | 203-205 |
| 19 | 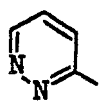 | H | 4-NHCOCOOH Ethanolamine salt | 199-201 |
| 20 | 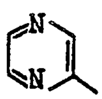 | H | 4-$NHCOCOOC_2H_5$ | 165-166 |
| 21 | 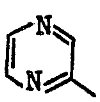 | H | 4-NHCOCOOH Sodium salt | >300 |
| 22 | 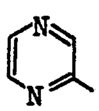 | H | 3-$NHCOCOOC_2H_5$ | 131-133 |
| 23 | 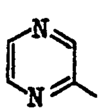 | H | 3-NHCOCOOH Ethanolamine salt | 205-206 |
| 24 | 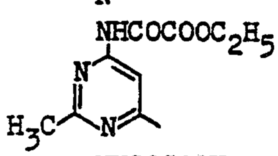 | H | 4-$NHCOCOOC_2H_5$ | 203-205 |
| 25 | 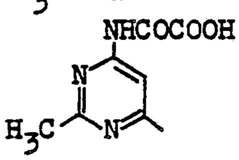 | H | 4-NHCOCOOH diethanolamine hydrate salt | 274-275 |

| Example No. | A | $R_2$ | $NHCOCOOR_1$ | m.p. °C |
|---|---|---|---|---|
| 26 | 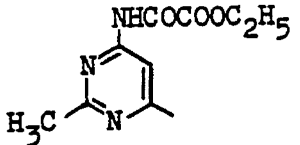 | H | 3-$NHCOCOOC_2H_5$ hemihydrate | 197-199 |
| 27 | 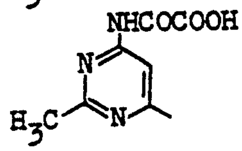 | H | 3-NHCOCOOH di-tromethane salt | 197-199 |
| 28 | 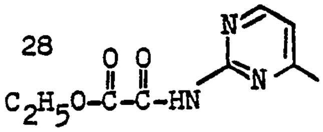 | H | 3-$NHCOCOOC_2H_5$ | 199-201 |
| 29 | 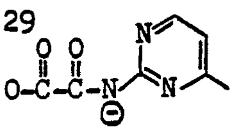 | H | 3-$\overset{\ominus}{N}$-COCO$\overset{\ominus}{O}$ tetra-sodium salt dihydrate | >350 |
| 30 | 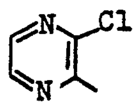 | H | 4-$NHCOCOOC_2H_5$ | |
| 31 | 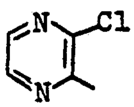 | H | 4-NHCOCOOH Ethanolamine salt | |
| 32 | 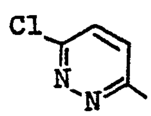 | H | 4-$NHCOCOOC_2H_5$ | |
| 33 | 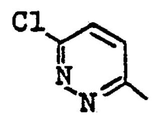 | H | 4-NHCOCOOH Ethanolamine salt | |

The compounds of the present invention, that is, those embraced by formula I above and their non-toxic, pharmacologically acceptable salts, have useful pharmacodynamic properties. More particularly, they exhibit immunological, anti-inflammatory and anti-allergic activities in warm-blooded animals such as rats, and are therefore useful for the treatment of allergic diseases such as allergic asthma, rhinitis, conjunctivitis, hay fever, urticaria, food allergies and the like.

Representative compounds of the present invention were tested comparatively with cromoglycate to determine in vivo anti-allergic activity.

The anti-allergic properties were ascertained in rats by the Passive Cutaneous Anaphylaxis test (PCA) essentially as described by Goose and Blair (Immunology 16: 749—760, 1969). Rat serum was diluted so that the skin reactions with diameters between 10 and 15 mm in unsensitized rats were produced. The PCA test was performed in duplicate by injecting 0.1 ml of this antiserum dilution on each side of the shaved back of rats. Rats so treated were injected intravenously (i.v.) twenty-four hours later with 0.02 mg ovalbumin in 0.5 ml of 1% Evans Blue solution within five minutes after intrevenous administration or 30 minutes after oral administration of the test compounds. Thirty minutes after the ovalbumin challenge the rats were killed by $CO_2$-asphyxiation, and the skin was reflected. The diameters, in millimeters, of the blued areas were measured and the mean diameter was determined. The circular area was calculated, and the mean area in square millimeters of the control group was considered as 100%, and the results of the compound test groups were expressed as a percentage change from these control values. From dose response curves the dose reducing the size of the blued area by 50% ($ED_{50}$) was estimated.

The following table shows the results of this test for a few representative specific compounds of the present invention:

| Compound of Example No. | Nomenclature | $ED_{50}$ mg/kg,p.o. |
|---|---|---|
| | Cromoglycate | Inactive |
| 1 | Ethyl 2-(4-pyrimidinyl)ethenylphenyl-4 oxamate | 1.4 |
| 4 | Ethyl 2-(4-pyrimidinyl)ethenylphenyl-3 oxamate | 2.8 |
| 5 | 2-(4-Pyrimidinyl)ethenylphenyl-3 oxamic acid ethanolamine salt | 12.3 |
| 7 | 2-(4-Pyrimidinyl)ethenylphenyl-2 oxamic acid ethanolamine salt | 8 |
| 8 | Ethyl 2-(4-pyrimidinyl-ethenyl-2-hydroxyphenyl-4 oxamate | 4.5 |
| 9 | Ethyl 2-(4-pyrimidinyl)ethenyl-5-chlorophenyl-3 oxamate | 2 |
| 12 | Diethyl 2-(4-pyrimidinyl)ethenylphenyl-2,4-dioxamate | 2 |
| 13 | Ethyl 2-(2,6-diethoxypyrimidin-4-yl)ethenyl-phenyl-4 oxamate | 30 |
| 14 | Ethyl 2-(2-methyl-6-aminopyrimidin-4-yl)-ethenylphenyl-4 oxamate | 10 |
| 15 | Ethyl 2-(6-methylpyrimidin-4-yl)ethenylphenyl-4 oxamate | 1.1 |
| 18 | Ethyl 2-(3-pyridazinyl)ethenylphenyl-4 oxamate | 1 |
| 20 | Ethyl 2-(2-pyrazinyl)ethenylphenyl-4 oxamate | 4.6 |
| 21 | 2-(2-Pyrazinyl)ethenylphenyl-4 oxamic acid sodium salt | 10 |
| 22 | Ethyl 2-(2-pyrazinyl)ethenylphenyl-3 oxamate | 4 |
| 23 | 2-(2-Pyrazinyl)ethenylphenyl-3 oxamic acid ethanolamine salt | $<1$ |
| 24 | Ethyl 2-(2-methyl-6-ethoxyoxalylaminopyrimidin-4-yl)-ethenylphenyl-4 oxamate | 0.3 |
| 25 | 2-(2-Methyl-6-carboxycarbonylaminopyrimidin-4-yl)-ethenylphenyl-4 oxamic acid hydrate diethanolamine salt | 4.0 |
| 3 | Ethyl 2-(2-ethoxyoxalylaminopyrimidin-4-yl)-ethenylphenyl-4 oxamate | 8 |

For pharmaceutical purposes the compounds according to the present invention are administered to warm-blooded animals topically, perorally, parenterally, rectally or by the respiratory route as active ingredients in customary pharmaceutical compositions, that is, compositions consisting essentially of an inert pharmaceutical carrier and an effective amount of the active ingredient.

When the compounds of the formula I are given by the oral route, they may be formulated in the form of syrups, tablets, capsules, pills and the like. Preferably, the compositions are in unit dosage form, or in a

form in which the patient can administer to himself a single dose. When the composition is in the form of a tablet, powder or lozenge, any pharmaceutical carrier suitable for formulating solid compositions may be used. Examples of such carriers are various starches, lactose, glucose, sucrose, cellulose, dicalcium phosphate, and chalk. The composition may also be in the form of an ingestible capsule (for example of gelatin) containing the compound; or in the form of a syrup, a liquid solution or a suspension. Suitable liquid pharmaceutical carriers include ethyl alcohol, glycerin, saline, water, propylene glycol or sorbitol solution, which may be compounded with flavoring or coloring agents to form syrups.

The compounds of this invention may also be administered by other than the oral route. In accordance with routine pharmaceutical procedure, the compositions may be formulated, for example, for rectal administration as a suppository or for presentation in an injectable form in an aqueous or non-aqueous solution, suspension or emulsion in a pharmaceutically acceptable liquid, such as sterile, pyrogen-free water of a parenterally acceptable oil or a mixture of liquids, which may contain bacterostatic agents, antioxidants, preservatives, buffers, or other solutes to render the solution isotonic with the blood, thickening agents, suspending agents or other pharmaceutically acceptable additives. Such forms will be presented in unit dose forms such as ampules or disposable injection devices or in multi-dose vials such as a bottle from which the appropriate dose may be withdrawn, or in solid form or concentrate which can be used to prepare an injectable formulation.

Compounds of this invention may also be suitably presented for administration to the respiratory tract as an aerosol or solution for a nebulizer, or as a microfine powder for insufflation, alone or in combination with an inert carrier such as lactose. In such a case the particles of active compounds suitably have diameters of less than 20 microns, preferably less than 10 microns. Where appropriate, small amounts of other anti-allergics, anti-asthmatics and bronchodilators, for example, sympathomimetic amines such as isoprenaline, isoetharine, metaproterenol, salbutamol, phenylephrine, fenoterol and ephedrine; xanthine derivatives such as theophylline and aminophylline; corticosteroids such as prednisolone and adrenal stimulants such as ACTH may be included.

Compounds of this invention may also be presented as an ointment, cream, lotion, gel, aerosol or solution for topical application to the skin, nose, or eye.

In any of the foregoing formulations, a suitable dosage unit may contain from 1 to 500 mg of active ingredient. The effective dose of compounds of this invention depends on the particular compound employed, the condition of the patient and on the frequency and route of administration, but in general it is in the range of from 0.01 mg/kg to 100 mg/kg body weight.

As is common practice, the compositions will usually be accompanied by written or printed directions for use in the medical treatment concerned, in this case as an anti-allergic agent for the prophylaxis and treatment of for example, asthma, hay-fever, rhinitis or allergic eczema.

For the preparation of pharmaceutical compositions, the compounds of general formula (I) are mixed in the usual way with appropriate pharmaceutical carrier substances and aroma, flavoring and coloring materials and formed, for example, into tablets or capsules or, with the addition of appropriate adjuvants, suspended or dissolved in water or in an oil, for example corn oil.

The compounds of this invention can be administered orally and parenterally in liquid or solid form. As injection medium, it is preferred to use water which contains the stabilizing agents, solubilizing agents and/or buffers conventionally used for injection solutions. Additives of this type include, for example, tartrate, citrate and acetate buffers, ethanol, propylene glycol, polyethylene glycol, complex formers (such as EDTA), antioxidants (such as sodium bisulfate, sodium metabisulfate or ascorbic acid), high-molecular-weight polymers (such as liquid-polyethylene oxides) for viscosity regulation, and polyethylene derivatives of sorbitol anhydrides.

Preservatives may also be added, if necessary, such as benzoic acid, methylene propylparaben, benzalkonium chloride or other quaternary ammonium compounds.

Solid carrier materials which can be used include, for example, starch, lactose, mannitol, methyl cellulose, microcrystalline cellulose, talcum, pyrogenic silica, dicalcium phosphate, and high-molecular-weight polymers (such as polyethylene glycol).

Compositions suitable for oral administration can, if desired, contain flavoring and/or sweetening agents. For topical administration, the compounds of the present invention can also be used in the form of powders or ointments, for which purpose they are mixed with, for example, powdered, physiologically compatible diluents or conventional ointment bases.

The following examples illustrate a few pharmaceutical dosage unit compositions comprising a compound of the present invention as an active ingredient and represent the best modes contemplated of using the invention. The parts are parts by weight unless otherwise specified.

Example 34

*Tablets*

The tablet composition is compounded from the following ingredients:

| | | |
|---|---|---|
| Ethyl 2-(2-methyl-6-ethoxyoxalylpyrimidin-4-yl)-ethenylphenyl-4-oxamate | | 0.010 parts |
| Stearic acid | | 0.010 parts |
| Dextrose | | 1.890 parts |
| | Total | 1.910 parts |

Preparation:

The ingredients are admixed in conventional manner, and the mixture is compressed into 1.91 g-tablets, each of which is an oral dosage unit composition containing 10 mg of the active ingredient.

Example 35

*Ointment*

The ointment composition is compounded from the following ingredients:

| | | |
|---|---|---|
| Ethyl 2-(3-pyridazinyl)ethenylphenyl-4 oxamate | | 2.000 parts |
| Fuming hydrochloric acid | | 0.01 parts |
| Sodium pyrosulfite | | 0.050 parts |
| Mixture (1:1) of cetyl alcohol and stearyl alcohol | | 20.000 parts |
| White vaseline | | 5.000 parts |
| Synthetic bergamot oil | | 0.075 parts |
| Distilled water | q.s.ad | 100.000 parts |

Preparation:

The ingredients are uniformly blended in conventional manner into an ointment, 100 g of which contain 2.0 g of the active ingredient.

Example 36

*Inhalation aerosol*

The aerosol composition is compounded from the following ingredients:

| | | |
|---|---|---|
| Ethyl 2-(4-pyrimidinyl)ethenylphenyl-4 oxamate | | 1.00 parts |
| Soybean lecithin | | 0.20 parts |
| Propellant gas mixture (Freon 11, 12 and 14) | q.s. | 100.00 parts |

Preparation:

The ingredients are compounded in conventional manner, and the composition is filled into aerosol containers with a metering valve which releases 0.5 to 2.0 mg of active ingredient per actuation of the valve.

Example 37

*Hypodermic solution*

The solution is compounded from the following ingredients:

| | | |
|---|---|---|
| 2-(2-Methyl-6-carboxycarbonylaminopyrimidin-4-yl)-ethenylphenyl-4 oxamate diethanolamine salt | | 5.0 parts |
| Sodium pyrosulfite | | 1.0 parts |
| Sodium salt of EDTA | | 0.5 parts |
| Sodium chloride | | 8.5 parts |
| Double-distilled water | q.s.ad | 1000.0 parts |

Preparation:

The individual ingredients are dissolved in a sufficient amount of double-distilled water, the solution is diluted to the indicated concentration with additional double-distilled water, the resulting solution is filtered until free from suspended particles, and the filtrate is filled under aseptic conditions into 1 ml-ampules which are subsequently sterilized and sealed. Each ampule contains 5 mg of the active ingredient.

Any one of the other compounds embraced by formula I or a non-toxic, pharmacologically acceptable acid addition salt thereof may be substituted for the particular active ingredient in Examples 36 through 39. Likewise, the amount of active ingredient in these illustrative examples may be varied to achieve the dosage unit range set forth above, and the amounts and nature of the inert pharmaceutical carrier ingredient may be varied to meet particular requirements.

**Claims for the Contracting States: BE CH DE FR GB IT LI LU NL SE**

1. A compound of the formula

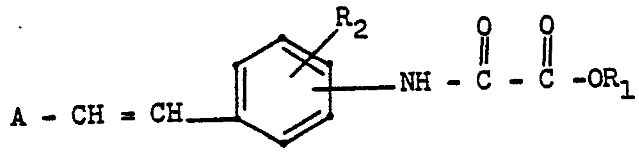

(I)

wherein

$R_1$ is hydrogen or alkyl of 1 to 4 carbon atoms;

$R_2$ is hydrogen, methyl, hydroxyl, alkoxy of 1 to 4 carbon atoms, di(alkyl of 1 to 2 carbon atoms)amino-(alkoxy of 1 to 4 carbon atoms) or —HN—CO—CO—$OR_1$;

A is

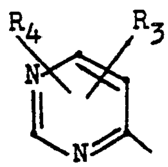 , 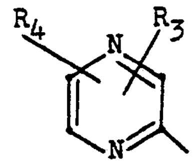 or 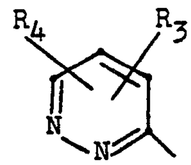

$R_3$ is hydrogen, methyl, alkoxy of 1 to 4 carbon atoms, hydroxyl, amino, alkanoyloxy of 1 to 2 carbon atoms, di(alkyl of 1 to 2 carbon atoms)amino-(alkoxy of 1 to 4 carbon atoms) or acetamido; and

$R_4$ is hydrogen, amino, alkoxy of 1 to 4 carbon atoms, halogen or —HN—CO—CO—$OR_1$;

or, when $R_1$ is hydrogen, non-toxic, pharmacologically acceptable salts thereof.

2. A compound of claim 1

wherein

$R_1$ is hydrogen or ethyl,

$R_2$ is hydrogen,

A is

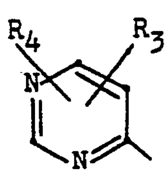 , 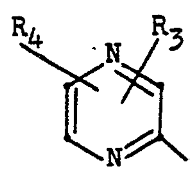 or 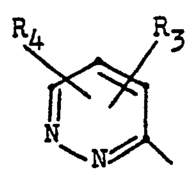

$R_3$ is hydrogen or methyl, and

$R_4$ is hydrogen, or —HN—CO—CO—$OR_1$,

or, when $R_1$ is hydrogen, a non-toxic, pharmacologically acceptable salts thereof.

3. A compound of claim 1 which is

diethyl-2-(4-pyrimidyl)ethenylphenyl-2,4-dioxamate ethyl 2-(2-methyl-6-ethoxyoxalylaminopyrimidin-4-yl)ethenylphenyl-4-oxamate;

the ethanolamine salt of 2-(2-pyrazinyl)ethenylphenyl-3-oxamic acid;

ethyl 2-(3-pyridazinyl)ethenylphenyl-4 oxamate;

ethyl 2-(6-methylpyrimidin-4-yl)ethenylphenyl-4 oxamate; or

ethyl 2-(4-pyrimidinyl)ethenylphenyl-4 oxamate.

4. A method of preparing a compound of formula I as defined in claim 1, or a non-toxic pharmaceutically acceptable salt thereof, characterized by reacting an amine of the formula

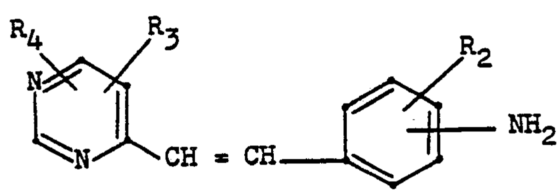 ,

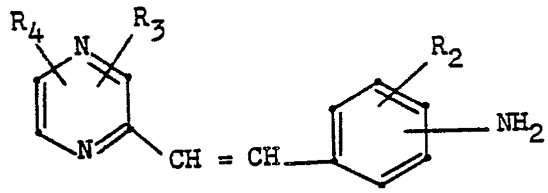

or

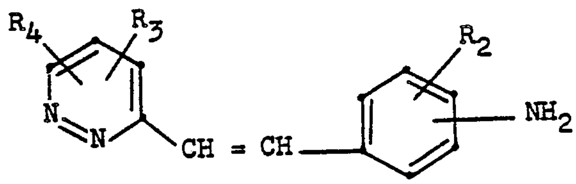

wherein $R_2$, $R_3$ and $R_4$ have the meanings defined in claim 1, with an oxalate halide or with a dialkyl oxalate, optionally followed by hydrolysis of the ester group and optionally by salt formation of the free acid.

5. An antiallergic and antiinflammatory pharmaceutical composition consisting essentially of an inert pharmaceutical carrier and an effective amount of a compound of claim 1.

6. The use of a compound in any one of claims 1 to 3 in the preparation of a pharmaceutical composition for the treatment of allergic and inflammatory reactions.

7. A compound of formula I or a non-toxic pharmaceutically acceptable salt thereof as defined in claim 1, for use in the treatment of allergic and inflammatory reactions in warm-blooded animals.

**Claims for the Contracting State: AT**

1. A method of preparing a compound of the formula

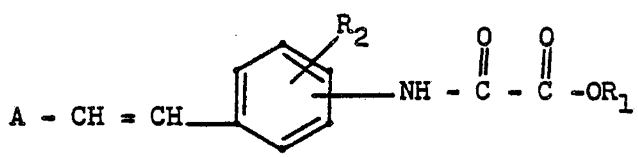

(I)

wherein

$R_1$ is hydrogen or alkyl of 1 to 4 carbon atoms;

$R_2$ is hydrogen, methyl, hydroxyl, alkoxy of 1 to 4 carbon atoms, di(alkyl of 1 to 2 carbon atoms)amino-(alkoxy of 1 to 4 carbon atoms) or —HN—CO—CO—$OR_1$;

A is

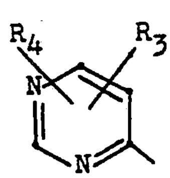

,

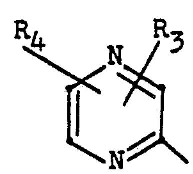

or

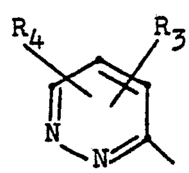

$R_3$ is hydrogen, methyl, alkoxy of 1 to 4 carbon atoms, hydroxyl, amino, alkanoyloxy of 1 to 2 carbon atoms, di(alkyl of 1 to 2 carbon atoms)amino-(alkoxy of 1 to 4 carbon atoms) or acetamido; and

$R_4$ is hydrogen, amino, alkoxy of 1 to 4 carbon atoms, halogen or —HN—CO—CO—$OR_1$;

or a non-toxic, pharmacologically acceptable salt thereof when $R_1$ is hydrogen, characterized by reacting an amine of the formula

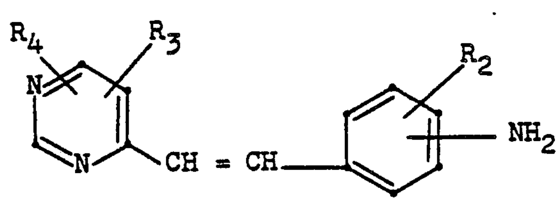

,

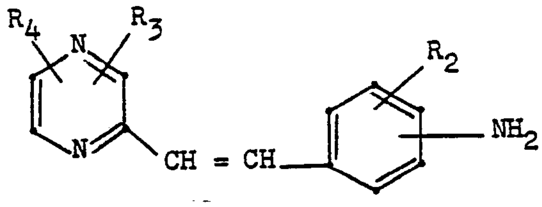

or

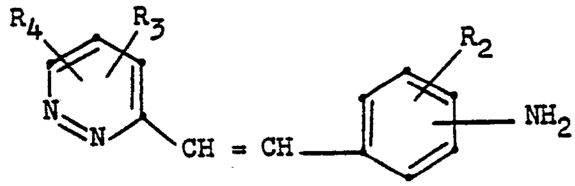

wherein $R_2$, $R_3$ and $R_4$ have the meanings previously defined, with an oxalate halide or with a dialkyl oxalate, optionally followed by hydrolysis of the ester group and optionally by salt formation of the free acid.

2. A method as defined in claim 1, characterized in that a compound of formula I is produced in which
$R_1$ is hydrogen or ethyl,
$R_2$ is hydrogen,
A is

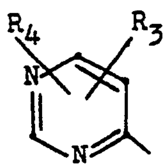 , 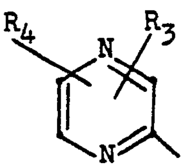 or 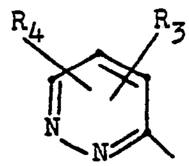

$R_3$ is hydrogen or methyl, and
$R_4$ is hydrogen, or —HN—CO—CO—$OR_1$.

3. A method as defined in claim 1 characterized in that there is produced
diethyl-2-(4-pyrimidyl)ethenylphenyl-2,4-dioxamate ethyl 2-(2-methyl-6-ethoxyoxalylaminopyrimidin-4-yl)ethenylphenyl-4-oxamate;
the ethanolamine salt of 2-(2-pyrazinyl)ethenylphenyl-3-oxamic acid;
ethyl 2-(3-pyridazinyl)ethenylphenyl-4 oxamate;
ethyl 2-(6-methylpyrimidin-4-yl)ethenylphenyl-4 oxamate or
ethyl 2-(4-pyrimidinyl)ethenylphenyl-4 oxamate.

4. A method of preparing a pharmaceutical composition which comprises mixing a compound of formula I or a non-toxic pharmaceutically acceptable salt thereof as defined in claim 1 with a pharmaceutically acceptable carrier substance.

5. A method of preparing a pharmaceutical composition which comprises mixing a compound of formula I or a non-toxic pharmaceutically acceptable salt thereof as defined in claim 1 when prepared by the process of claim 1, with a pharmaceutically acceptable carrier substance.

**Patentansprüche für die benannten Vertragsstaaten: BE CH DE FR GB IT LI LU NL SE**

1. Eine Verbindung der Formel

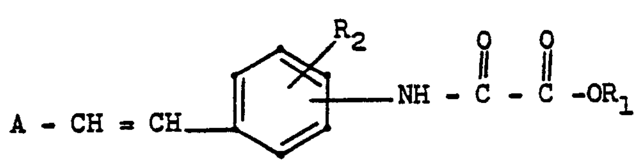 (I)

worin
$R_1$ ein Wasserstoffatom oder eine Alkylgruppe mit 1 bis 4 Kohlenstoffatomen;
$R_2$ ein Wasserstoffatom, eine Methyl-, Hydroxyl-, eine Alkoxygruppe mit 1 bis 4 Kohlenstoffatomen,

eine Dialkylamino-alkoxygruppe mit 1 bis 2 Kohlenstoffatomen im Alkylrest und 1 bis 4 Kohlenstoffatomen im Alkoxyrest oder die Gruppe —HN—CO—CO—$OR_1$ darstellen

A für die Gruppen

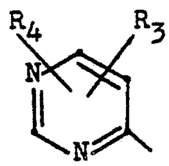 , 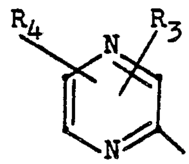 oder 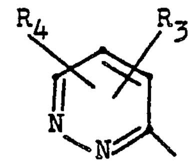

$R_3$ ein Wasserstoffatom, die Methyl- oder eine Alkoxygruppe mit 1 bis 4 Kohlenstoffatomen, die Hydroxyl- oder Aminogruppe, eine Alkanoyloxygruppe mit 1 oder 2 Kohlenstoffatomen, eine Dialkylamino-alkoxygruppe mit 1 oder 2 Kohlenstoffatomen im Alkylrest und 1 bis 4 Kohlenstoffatomen im Alkoxyrest oder die Acetamidogruppe und

$R_4$ ein Wasserstoffatom, die Aminogruppe, eine Alkoxygruppe mit 1 bis 4 Kohlenstoffatomen, ein Halogenatom oder die Gruppe —HN—CO—CO—$OR_1$ darstellen;

oder, wenn $R_1$ ein Wasserstoffatom ist, ihre nicht toxischen pharmakologisch verträglichen Salze.

2. Eine Verbindung gemäß Anspruch 1 worin

$R_1$ ein Wasserstoffatom oder eine Ethylgruppe,

$R_2$ ein Wasserstoffatom,

A die Gruppen

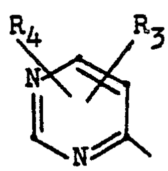 , 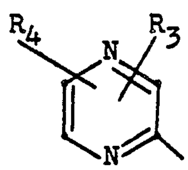 oder 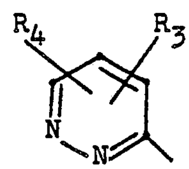

$R_3$ ein Wasserstoffatom oder eine Methylgruppe, und

$R_4$ ein·Wasserstoffatom oder die Gruppe —HN—CO—CO—$OR_1$ bedeuten,

oder, wenn $R_1$ ein Wasserstoffatom ist, ihre nichttoxischen pharmakologisch verträglichen Salze.

3. Als Verbindungen gemäß Anspruch 1 die Verbindungen:

Diethyl-2-(4-pyrimidyl)ethenylphenyl-2,4-Dioxamat, N-[4-[2-(2-Methyl-6-ethoxyoxalylaminopyrimidin-4-yl)ethenylphenyl]]-oxalsäuremonoethylesteramid;

das Ethanolaminsalz des N-[3-[2-Pyrazinyl)ethenylphenyl]]oxalsäuremonoamids;

N-[4-[2-(3-Pyridazinyl)ethenylphenyl]]oxalsäuremonoethylesteramid;

N-[4-[2-(6-Methylpyrimidin-4-yl)ethenylphenyl]]oxalsäuremonoethylesteramid; oder

N-[4-[2-(4-Pyrimidinyl)ethenylphenyl]]oxalsäuremonoethylesteramid.

4. Verfahren zur Herstellung von Verbindungen der Formel I gemäß Anspruch 1 oder von deren nichttoxischen, pharmazeutisch verträglichen Salzen, dadurch gekennzeichnet, daß ein Amin der Formeln

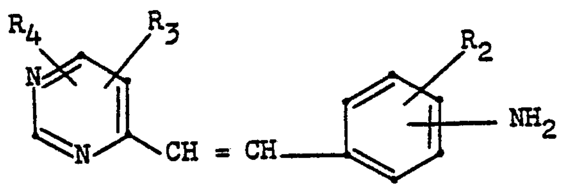 ,

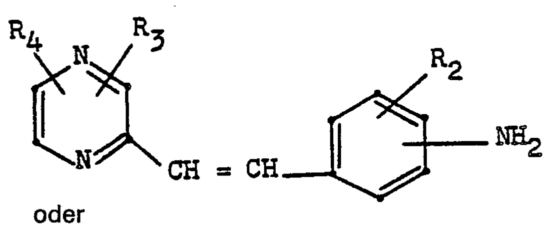

oder

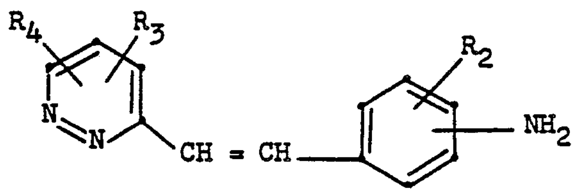

worin $R_2$, $R_3$ und $R_4$ die in Anspruch 1 genannten Bedeutungen haben, mit einem Oxalsäurehalid oder mit einem Dialkyloxalat umgesetzt wird, gewünschtenfalls unter anschließender Hydrolyse der Estergruppe und gewünschtenfalls unter anschließener Überführung der freien Säure in ihre Salze.

5. Antiallergische und entzündungshemmende pharmazeutische Zubereitungsformen, die im wesentlichen aus einem inerten pharmazeutischen Trägerstoff und einer wirksamen Menge einer Verbindung gemäß Anspruch 1 bestehen.

6. Die Verwendung einer Verbindung gemäß einem der Ansprüche 1 bis 3 zur Herstellung einer pharmazeutischen Zubereitungsform zur Behandlung von allergischen und entzündlichen Reaktionen.

7. Eine Verbindung der Formel I oder ein nichttoxisches pharmazeutisch verträgliches Salz davon, wie in Anspruch 1 dargestellt, zur Verwendung in der Behandlung von allergischen und entzündlichen Reaktionen in warmblütigen Tieren.

**Patentansprüche für den benannten Vertragsstaat: AT**

1. Verfahren zur Herstellung von Verbindungen der Formel

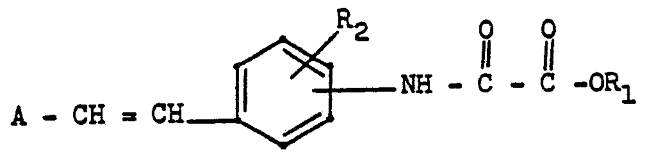

(I)

worin

$R_1$ ein Wasserstoffatom oder eine Alkylgruppe mit 1 bis 4 Kohlenstoffatomen;

$R_2$ ein Wasserstoffatom, eine Methyl-, Hydroxyl-, eine Alkoxygruppe mit 1 bis 4 Kohlenstoffatomen, eine Dialkylamino-alkoxygruppe mit 1 bis 2 Kohlenstoffatomen im Alkylrest und 1 bis 4 Kohlenstoffatomen im Alkoxyrest oder die Gruppe —HN—CO—CO—$OR_1$ darstellen

A für die Gruppen

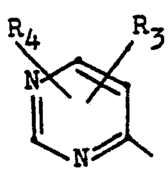

,

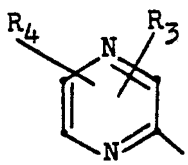

oder

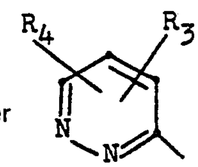

steht,

$R_3$ ein Wasserstoffatom, die Methyl- oder eine Alkoxygruppe mit 1 bis 4 Kohlenstoffatomen, die Hydroxyl- oder Aminogruppe, eine Alkanoyloxygruppe mit 1 oder 2 Kohlenstoffatomen, eine Dialkylamino-alkoxygruppe mit 1 oder 2 Kohlenstoffatomen im Alkylrest und 1 bis 4 Kohlenstoffatomen im Alkoxyrest oder die Acetamidogruppe und

$R_4$ ein Wasserstoffatom, die Aminogruppe, eine Alkoxygruppe mit 1 bis 4 Kohlenstoffatomen, ein Halogenatom oder die Gruppe —HN—CO—CO—$OR_1$ darstellen;

oder von nichttoxischen, pharmakologisch verträglichen Salzen hiervon, sofern $R_1$ ein Wasserstoffatom ist, dadurch gekennzeichnet, daß ein Amin der Formeln

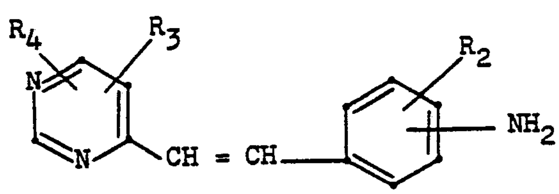

,

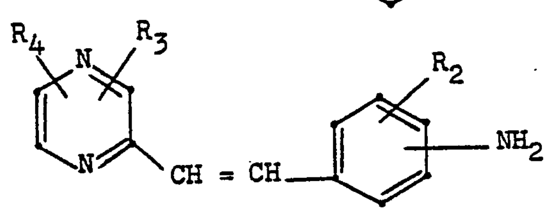

oder

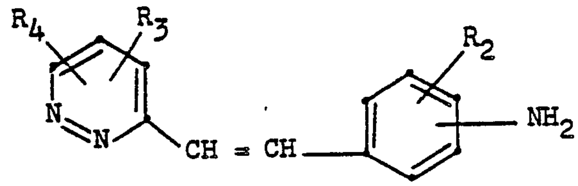

worin $R_2$, $R_3$ und $R_4$ die vorher genannten Bedeutungen haben, mit einem Oxalsäurehalid oder mit einem Dialkyloxalat umgesetzt wird, gewünschtenfalls unter anschließender Hydrolyse der Estergruppe und gewünschtenfalls unter anschließener Überführung der freien Säure in ihre Salze.

2. Verfahren gemäß Anspruch 1, dadurch gekennzeichnet, daß eine Verbindung der Formel I hergestellt wird, worin

$R_1$ ein Wasserstoffatom oder eine Ethylgruppe,

$R_2$ ein Wasserstoffatom,

A die Gruppen

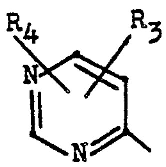
,
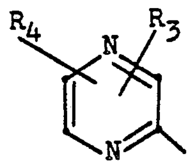
oder
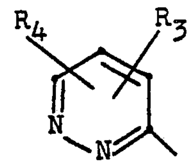

$R_3$ ein Wasserstoffatom oder eine Methylgruppe, und

$R_4$ ein Wasserstoffatom oder die Gruppe —HN—CO—CO—$OR_1$ bedeuten.

3. Verfahren gemäß Anspruch 1, dadurch gekennzeichnet, daß eine der folgenden Verbindungen hergestellt wird:

Diethyl-2-(4-pyrimidyl)ethenylphenyl-2,4-Dioxamat, N-[4-[2-(2-Methyl-6-ethoxyoxalylaminopyrimidin-4-yl)ethenylphenyl]]-oxalsäuremonoethylesteramid;

das Ethanolaminsalz des N-[3-[2-Pyrazinyl)ethenylphenyl]]oxalsäuremonoamids;

N-[4-[2-(3-Pyridazinyl)ethenylphenyl]]oxalsäuremonoethylesteramid;

N-[4-[2-(6-Methylpyrimidin-4-yl)ethenylphenyl]]oxalsäuremonoethylesteramid; oder

N-[4-[2-(4-Pyrimidinyl)ethenylphenyl]]oxalsäuremonoethylesteramid.

4. Verfahren zur Herstellung pharmazeutischer Zubereitungsformen, gekennzeichnet durch die Vermischung einer Verbindung der Formel I oder eines ihrer nichttoxischen, pharmazeutisch verträglichen Salze mit einer pharmazeutisch verträglichen Trägersubstanz.

5. Verfahren zur Herstellung pharmazeutischer Zubereitungsformen, gekennzeichnet durch die Vermischung einer Verbindung der Formel I oder eines ihrer nichttoxischen, pharmazeutisch verträglichen Salze, sofern sie nach dem Verfahren des Anspruchs 1 hergestellt sind, mit einer pharmazeutisch verträglichen Trägersubstanz.

**Revendications pour les Etats contractants: BE CH DE FR GB IT LI LU NL SE**

1. Composé répondant à la formule

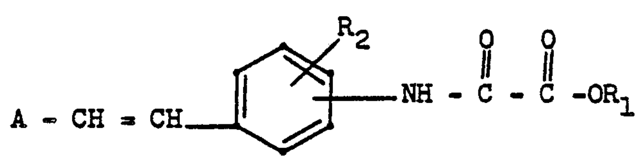
(I)

dans laquelle

$R_1$ est un atome d'hydrogène ou un groupe alkyle en $C_1$ à $C_4$;

$R_2$ est un atome d'hydrogène, un groupé méthyle, hydroxyle, alcoxy en $C_1$ à $C_4$, di(alkyle en $C_1$ ou $C_2$) amino-(alcoxy en $C_1$ à $C_4$) ou —HN—CO—CO—$OR_1$;

A est

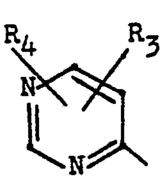
,
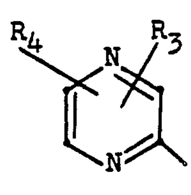
ou
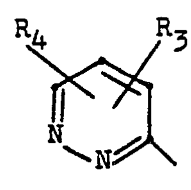

$R_3$ est un atome d'hydrogène, un groupe méthyle, alcoxy en $C_1$ à $C_4$, hydroxyle, amino, alcanoyloxy en $C_1$ à $C_2$, di(alkyle en $C_1$ à $C_2$)-amino-(alcoxy en $C_1$ à $C_4$) ou acétamido; et

$R_4$ est un atome d'hydrogène, un groupe amino, alcoxy en $C_1$ à $C_4$, un atome d'halogène ou un groupe —HN—CO—CO—$OR_1$;

ou, lorsque $R_1$ est un atome d'hydrogène, un de ses sels non toxiques, pharmacologiquement acceptables.

2. Composé suivant la revendication 1, dans laquelle

$R_1$ est un atome d'hydrogène ou un groupe éthyle,

$R_2$ est un atome d'hydrogène,

A est

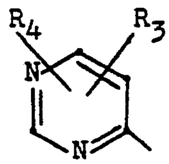
,
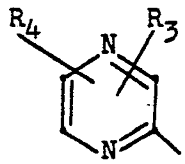
ou
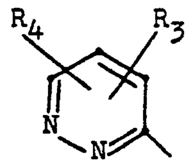

$R_3$ est un atome d'hydrogène ou un groupe méthyle, et

$R_4$ est un atome d'hydrogène ou un groupe —HN—CO—CO—$OR_1$,

ou, lorsque $R_1$ est un atome d'hydrogène, un de ses sels non toxiques, pharmacologiquement acceptables.

3. Composé de la revendication 1, qui est

le 2-(4-pyrimidyl)éthénylphényl-2,4 dioxamate de diéthyle;

le 2-(2-méthyl-6-éthoxyoxalylaminopyrimidin-4-yl)-éthénylphényl-4 oxamate d'éthyle;

le sel d'éthanolamine de l'acide 2-(2-pyrazinyl)éthénylphényl-3 oxamique;

le 2-(3-pyridazinyl)éthénylphényl-4 oxamate d'éthyle;

le 2-(6-méthylpyrimidin-4-yl)éthénylphényl-4 oxamate d'éthyle; ou

le 2-(4-pyrimidinyl)éthénylphényl-4 oxamate d'éthyle.

4. Procédé de préparation d'un composé répondant à la formule I tel que défini dans la revendication 1, ou d'un de ses sels non toxiques, pharmaceutiquement acceptables, caractérisé en ce qu'on fait réagir une amine répondant à la formule

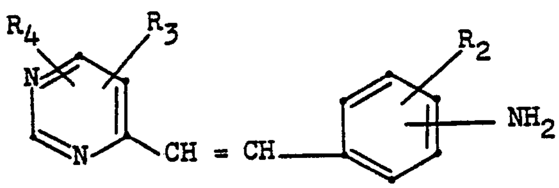
,
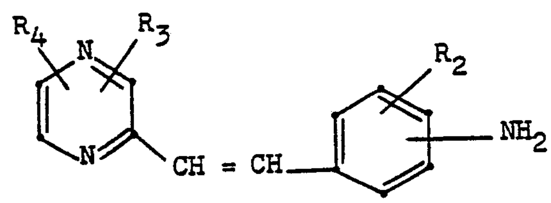
ou
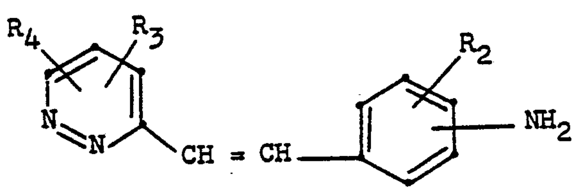

dans laquelle $R_2$, $R_3$ et $R_4$ ont les significations définies dans la revendication 1, avec un halogénure d'oxalyle ou avec un oxalate de dialkyle, après quoi on hydrolyse si on le désire le groupe ester et on forme si on le désire un sel de l'acide libre.

5. Composition pharmaceutique anti-allergique et anti-inflammatoire essentiellement constituée d'un support pharmaceutique inerte et d'une quantité efficace d'un composé suivant la revendication 1.

6. Utilisation d'un composé suivant l'une quelconque des revendications 1 à 3, dans la préparation d'une composition pharmaceutique pour le traitement de réactions allergiques et inflammatoires.

7. Composé répondant à la formule I ou un de ses sels non toxiques pharmaceutiquement acceptables, tels que définis dans la revendication 1, pour l'utilisation dans le traitement des réactions allergiques et inflammatoires chez les animaux à sang chaud.

**Revendications pour l'Etat contractant: AT**

1. Procédé de préparation d'un composé répondant à la formule

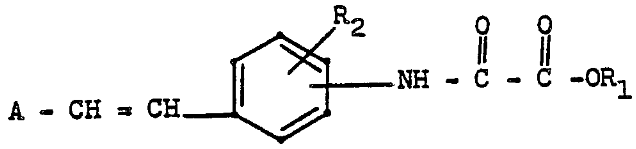 (I)

dans laquelle

$R_1$ est un atome d'hydrogène ou un groupe alkyle en $C_1$ à $C_4$;

$R_2$ est un atome d'hydrogène, un groupé méthyle, hydroxyle, alcoxy en $C_1$ à $C_4$, di(alkyle en $C_1$ ou $C_2$)-amino-(alcoxy en $C_1$ à $C_4$) ou un groupe —HN—CO—CO—$OR_1$;

A est

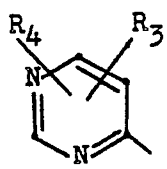 , 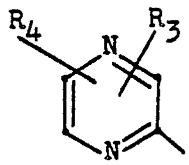 ou 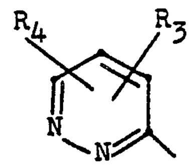

$R_3$ est un atome d'hydrogène, un groupe méthyle, alcoxy en $C_1$ à $C_4$, hydroxyle, amino, alcanoyloxy en $C_1$ à $C_2$, di(alkyle en $C_1$ à $C_2$)-amino-(alcoxy en $C_1$ à $C_4$) ou acétamido; et

$R_4$ est un atome d'hydrogène, un groupe amino, alcoxy en $C_1$ à $C_4$, un atome d'halogène ou un groupe —HN—CO—CO—$OR_1$;

ou un de ses sels non toxiques, pharmacologiquement acceptables, lorsque $R_1$ est un atome d'hydrogène, caractérisé en ce qu'on fait réagir une amine répondant à la formule

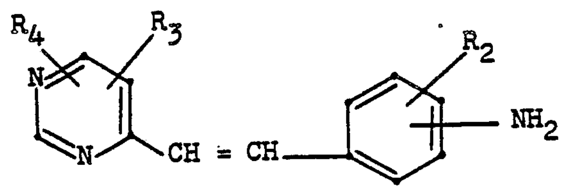 ,

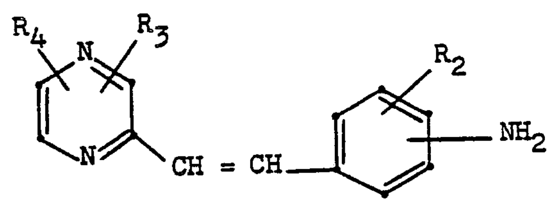

ou

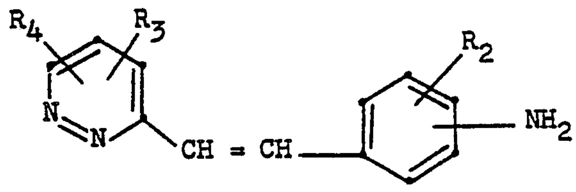

dans laquelle $R_2$, $R_3$ et $R_4$ ont les significations précédemment définies, avec un halogénure d'oxalyle ou avec un oxalate de dialkyle, après quoi on hydrolyse si on le désire le groupe ester et on forme si on le désire le sel de l'acide libre.

2. Procédé suivant la revendication 1, caractérisé en ce qu'on prépare un composé répondant à la formule I dans laquelle

$R_1$ est un atome d'hydrogène ou un groupe éthyle,

$R_2$ est un atome d'hydrogène,

A est

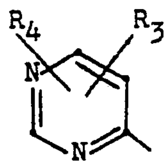 , 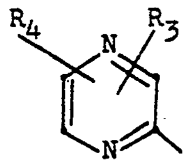 ou 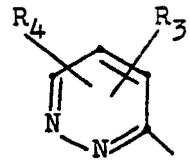

$R_3$ est un atome d'hydrogène ou un groupe méthyle, et
$R_4$ est un atome d'hydrogène ou un groupe —HN—CO—CO—$OR_1$.

3. Procédé suivant la revendication 1, caractérisé en ce qu'on prépare
le 2-(4-pyrimidyl)éthénylphényl-2,4 dioxamate de diéthyle;
le 2-(2-méthyl-6-éthoxyoxalylaminopyrimidin-4-yl)-éthénylphényl-4 oxamate d'éthyle;
le sel d'éthanolamine de l'acide 2-(2-pyrazinyl)éthénylphényl-3 oxamique;
le 2-(3-pyridazinyl)éthénylphényl-4 oxamate d'éthyle;
le 2-(6-méthylpyrimidin-4-yl)éthénylphényl-4 oxamate d'éthyle; ou
le 2-(4-pyrimidinyl)éthénylphényl-4 oxamate d'éthyle.

4. Procédé de préparation d'une composition pharmaceutique qui comprend le mélange d'un composé répondant à la formule I ou d'un de ses sels non toxiques pharmaceutiquement acceptables tels que définis dans la revendication 1 avec une substance de support pharmaceutique acceptable.

5. Procédé de préparation d'une composition pharmaceutique qui comprend le mélange d'un composé répondant à la formule I ou d'un de ses sels non toxiques, pharmaceutiquement acceptables tels que définis dans la revendication 1, lorsqu'il est préparé par le procédé de la revendication 1, avec une substance de support pharmaceutiquement acceptable.